Europäisches Patentamt

European Patent Office

Office européen des brevets

(11) Publication number: 0 332 600
A1

# EUROPEAN PATENT APPLICATION

(21) Application number: 89850083.0

(22) Date of filing: 10.03.89

(51) Int. Cl.⁴: A 61 K 9/18
A 61 K 47/00, A 61 K 31/785

(30) Priority: 11.03.88 DE 3808191

(43) Date of publication of application:
13.09.89 Bulletin 89/37

(84) Designated Contracting States: ES GR

(71) Applicant: Aktiebolaget Hässle
Kärragatan 5
S-431 83 Mölndal (SE)

(72) Inventor: Sunderdiek, Rainer
Unterhainstrasse 19
D-8750 Aschaffenburg (DE)

(74) Representative: Linderoth, Margareta et al
AB Astra Patent and Trademark Department
S-151 85 Södertälje (SE)

The title of the invention has been amended (Guidelines for Examination in the EPO, A-III, 7.3).

(54) Pharmaceutical compositions containing cholestyramine.

(57) A cholestyramine containing pharmaceutical composition in the form of an aqueous suspension having a viscosity of from 100 to 400 mPas, in which an anionized substance, in particular carboxymethyl cellulose, is bound to the cholestyramine particles to improve the taste, which also contains a salt or ester of sorbic acid as a preservative and optionally also contains aroma constituents.

EP 0 332 600 A1

**Description**

## NEW PHARMACEUTICAL COMPOSITION

The invention relates to a new cholestyramine containing composition.

Cholestyramine is a basic anion exchange resin on the basis of styrene-divinylbenzene with quarternary ammonium groups which are the carriers of the exchangeable anions. This substance forms sparingly soluble to insoluble precipitation complexes with large molecular anions. For that reason cholestyramine is used for the binding of bile acids on bile stagnation and hypercholesterolemia and hyperbilirubinemia. Furthermore, cholestyramine is known as a lipid lowering agent.

The raw material, which is obtained by emulsion polymerization, can be ground to very small particles (5 to 150 microns) in a special process with the aid of carbon dioxide snow. In this way a very large active surface can be obtained, which in addition to the amount of exchangeable groups is essential to the extent of binding of the bile acids.

Cholestyramine is hitherto most often included in a powdered administration form, which has an extremely unpleasant taste and leaves the impression of having eaten sea sand. This is attributed to the cationic character of cholestyramine which gives the active substance a marked affinity to mucous membranes and a high degree of adhesiveness to mucous membranes. This manifests itself on oral taking of the powder per se or of mixtures in extremely unpleasant taste conditions and impairs the compliance with the composition considerably. An oral suspension is described for example in US patent 3,974,272, which teaches combining cholestyramine with a modified gum. Although pharmaceutically effective, these known compositions are still very undesirable to drink, since the compositions form a gritty coating on the surface inside of the mouth. Other oral preparations in the form of dry water-dispersable granules or tablets are described in EP 278 464.

The problem of the invention is to provide a cholestyramine containing pharmaceutical composition, which represents a simply usable, i.e. which can be taken per se, and good tasting administration form.

This problem is solved by the characterizing features of claim 1.

The subclaims are characterizing further developments of the invention.

By means of the invention a ready-to-use administration form is provided which before administration does not need to be mixed with any solution, for instance fruit juice such as orange juice and the like in order to form an administration form which is fairly agreeably drinkable to the patient. By means of the suspension according to the invention an improvement is obtained with respect to the administration form as well as with respect to the taste in comparison with the known administration forms. By means of the addition of an anionic substance bound to the cholestyramine particles the taste of the ready-for-use suspension is improved considerably and can be further improved by means of the addition of suitable aromas such as, for instance, apricot aroma or the like. Low molecular anionic carboxymethyl cellulose (sodium carboxymethyl cellulose) is preferably used as the anionized substance. Besides carboxymethyl cellulose also other anionic polymers such as, for instance, carboxymethyl starches (salts), alginates, pectins, xanthan gums, galactomannans, carrageens, polyacrylates are contemplated. The binding capacity of cholestyramine for bile acids is not destroyed by the superficial binding of comparatively high molecular molecules of selected anionic polymers, in particular polysaccharides, e.g. certain types of Na-carboxymethyl cellulose, which is deposited on the surface of the cholestyramine particles by adsorption. This applies within a wide pH range (neutral and acid). This was established with the activity test according to USP XXI.

In view of the fact that cholestyramine is an ion exchange resin there is the risk when selecting inappropriate preservatives, that these are losing their preserving activity, since cholestyramine will bind charged preservative molecules due to its ability to exchange ions. Suitable preservatives are in spite of their salt character sorbic acid or esters thereof, in particular sodium, potassium, calcium and here preferably potassium sorbate. Other substances which are active in lower concentrations, e.g. parabens are bound so strongly that the demands for preserving activity according to DAB 9 is not complied with any more.

The carboxymethyl cellulose which is added to improve the taste of the composition is adsorbed comparatively slowly on the surface of the cholestyramine particles so that an equilibrium is established only after standing for a certain period (at least over night). This equilibrium, however, can also appear only within a few days (five to seven days) during which time a decrease in viscosity to a well flowable and pumpable suspension is observed. This decrease in viscosity can be explained by an increasing screening off of the resin particles, whereby interactions with each other, which, for instance could lead to agglomeration, are increasingly obstructed and prevented. The particle size of the cholestyramine particles influences the viscosity of the final suspension to a small extent, since the cholestyramine particles can be prepared with a very narrow and uniform particle distribution and size and are commercially available so. A preferred particle size lies between 10 and 40 microns.

The after-thickening of the suspension which occurs during the preparation disappears after standing for a certain time so that the suspension is flowable to a sufficient extent and exhibits a viscosity of 100 to 400 mPas.

The final homogenous suspension, which has a weakly yellowish colour, can be poured into ready-to-use packs, e.g. aluminium bags, which consist of a laminate combination (polyester, aluminium, polyethylene) or in bottles.

For the preparation of the suspension, purified water and propylene glycol in a ratio of preferably 6.5:1 are charged in a suitable mixing vessel, mixed and heated to 40°C. This ratio can also be selected within a range of from 5:1 to 8:1. Subsequently potassium sorbate and D-fructose are added and dissolved with stirring. Then sodium carboxymethyl cellulose is sprinkled into this solution with continuous stirring and is allowed to swell with continuous stirring. During the further course of production the cellulose slowly dissolves, at which the solution thickens distinctly. An aroma constituent, preferably apricot aroma, is subsequently introduced into the solution thus obtained and mixed therewith during stirring. Finally cholestyramine particles are introduced in portions with stirring and the use of a suitable homogenizer and mixed to visible homogenousness.

Subsequently the suspension is filled up with purified water to the desired final weight and is mixed once more with stirring - possibly with the use of the homogenizer. The final suspension is then deaerated with continuous stirring and with closed mixing vessel during at least 10 minutes by evacuation to a residual vacuum of about 100 mbar.

Then the suspension can be poured into the flat aluminium bags or bottles by means of an automatic filling and sealing device. If the ready-to-use suspension after the preparation thereof still is comparatively viscous and the pouring thereof made difficult for the above mentioned reasons, the mixing container should suitably be sealed and the ready-to-use suspension is filled only after standing for some time (at least over night).

In the following suitable compositions of the cholestyramine suspension are given:

| Compositions Nos. | (1) | (2) | (3) |
|---|---|---|---|
| | g/100 ml | g/100 ml | g/100 ml |
| Cholestyramine (dry substance) | 10.0 | 12.8 | 13.6 |
| D-Fructose | 10.0 | 13.9 | 14.6 |
| Propylene glycol | 5.0 | 9.4 | 9.8 |
| Sodium carboxymethyl cellulose | 1.0 | 1.65 | 1.7 |
| Apricot aroma | 0.015 | 0.017 | 0.18 |
| Potassium sorbate | 0.1 | 0.15 | 0.20 |

| Compositions Nos. | (4) | (5) | (6) |
|---|---|---|---|
| | g/100 ml | g/100 ml | g/100 ml |
| Cholestyramine (dry substance) | 14.035 | 15.2 | 18.0 |
| D-Fructose | 15.0 | 16.0 | 20.0 |
| Propylene glycol | 10.0 | 10.5 | 25.0 |
| Sodium carboxymethyl cellulose | 1.80 | 1.9 | 5.0 |
| Apricot aroma | 0.02 | 0.025 | 0.04 |
| Potassium sorbate | 0.27 | 0.28 | 0.3 |

| Composition No. 7 | |
|---|---|
| | g/100 ml |
| Cholestyramine | 10.8 |
| Saccharose | 10 |
| Propylene glycol | 8 |
| Potassium sorbate | 0.25 |
| Pectin | 0.8 |

| Composition No. 8 | |
|---|---|
| | g/100 ml |
| Cholestyramine | 12 |
| Saccharose | 10 |
| Propylene glycol | 10 |
| Potassium sorbate | 0.27 |
| Xanthan gum | 1.0 |

| Composition No. 9 | |
|---|---|
| | g/100 ml |
| Cholestyramine | 13 |
| Saccharose | 8 |
| Propylene glycol | 10 |
| Potassium sorbate | 0.26 |
| Sodium alginate | 1.2 |

By observing the given upper and lower limits for the components of the suspension it is ensured that the ready-to-use suspension is sufficiently flowable, pumpable and capable of being administered.

In view of the fact that the water content of cholestyramine can vary between 6% and 12%, it is necessary to consider the previously determined content of water when calculating the amount of cholestyramine to be used. The calculation of the amount of cholestyramine to be used is clear from the following formula:

$$\frac{100}{100-CW} \times m = \text{charge amount of cholestyramine}$$

(water-containing) in g per 100 ml batch size

CW = actual content of water in per cent

m = amount of cholestyramine in g per 100 ml batch calculated as dry substance.

The amount of the component water to be charged will be evident from the difference between the total weight of the batch and the sum of the other components.

The carboxymethyl cellulose which is preferably used as the anionic polymer, in particular sodium carboxymethyl cellulose, has an average substitution degree of 0.8, an average molecular weight of 80000 to 110000, preferably 90000, and a viscosity (2% in water) of 20 to 40 mPas, preferably 30 mPas.

A preferred administration form has the following properties:

Appearance: homogenous, slightly yellowish suspension.

Smell : of apricot aroma.

pH-value : 5.5 - 7.0.

Density : 1.088 g/ml.

Viscosity : 100 - 400 mPas.

Contents :

0.267 - 0.295 g bile acids binding/ml suspension (production specification)

4

0.239 - 0.295 g bile acids binding/ml suspension (duration specification)
2.43 - 2.97 mg potassium sorbate/ml suspension.
Packing :
Aluminium bag, laminate composition of 12 micron polyester, 9 micron aluminium, 50 micron polyethylene. 28.5 ml set volume for filling (respectively 31 g set weight for filling)
or
500 ml bottles of polyethylene which complies with the requirements according to foodstuff regulations. 513 ml set volume for filling (corresponding to 558 set weight for filling).

## Claims

1. A cholestyramine containing pharmaceutical composition, in which cholestyramine dry substance represents an amount of from 10 g/100 ml to 20 g/100 ml and an anionized substance (anionic polymer) is bound physically (adsorption) to the cholestyramine particles, characterized in that the composition has the form of an aqueous suspension having a viscosity of from 100 to 400 mPas, and that sorbic acid or a salt or ester thereof is added as a preservative.

2. Pharmaceutical composition according to claim 1, characterized in that aroma constituents are added as an additive to the suspension.

3. Pharmaceutical composition according to claim 2, characterized in a pH-value of from 4 to 8.

4. Pharmaceutical composition according to claim 1, characterized in that sodium, potassium or calcium sorbate is added as a preservative.

5. Pharmaceutical composition according to claim 4, characterized in that propylene glycol is added as a synergetic substance for the preservation.

6. Pharmaceutical composition according to claim 1, characterized in that the anionic substance is a polysaccharide.

7. Pharmaceutical composition according to claim 6, characterized in that the anionic substance is carboxymethyl cellulose.

8. Pharmaceutical composition according to claim 1, characterized in that the anionic substance is bound to the surface of the cholestyramine particles by adsorption.

9. Pharmaceutical composition according to claim 1, characterized in that the suspension also contains saccharose.

10. Pharmaceutical composition according to claim 9, characterized in that the suspension also contains D-fructose.

11. Pharmaceutical composition according to claim 2, characterized in that the aroma constituent is apricot aroma.

12. Pharmaceutical composition according to claim 1, characterized in that the suspension exhibits a density of from about 1 to 1.15 g/ml.

13. Pharmaceutical composition according to claim 1, characterized in that it contains, calculated on 100 ml aqueous suspension
10.0 to 18.0 g cholestyramine dry substance
10.0 to 20.0 g fructose
5.0 to 25.0 g propylene glycol
1.0 to 5.0 g carboxymethyl cellulose
0.01 to 0.04 g aroma
0.01 to 0.3 g salt of sorbic acid.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 251 369 (FORMENTI)<br>* Claims 1-3,8-10,15; page 3, lines 33,37-40; page 7, lines 39-42; page 8, line 15 * | 1-3,6-12 | A 61 K 9/18<br>A 61 K 47/00<br>A 61 K 31/785 |
| X | EP-A-0 190 826 (WARNER-LAMBERT)<br>* Claims 1,3-6,8,14,17; column 4, line 58; column 6, lines 5-12,45-47; column 8, lines 1-6; column 11, lines 35-40 * | 1-2,6-11 | |
| X | CHEMICAL ABSTRACTS, vol. 110, no. 14, 3rd April 1989, page 441, abstract no. 121382z, Columbus, Ohio, US; & DD-A-249 634 (VEB CHEMIEKOMBINAT BITTERFELD) 16-09-1987<br>* Abstract * | 1-2,6,8-11 | |
| D,X | US-A-3 974 272 (G.P. POLLI)<br>* Claims 1,3-7,9-11; column 2, lines 33-36 * | 1-2,6-11 | |
| D,P X | EP-A-0 278 464 (DOW)<br>* Claims 1,6,8-9; page 3, lines 23-30,40-43; page 4, lines 33-38; page 5, lines 12-13 * | 1-2,6-11 | TECHNICAL FIELDS SEARCHED (Int. Cl.4)<br><br>A 61 K |

The present search report has been drawn up for all claims

| Place of search | Date of completion of the search | Examiner |
|---|---|---|
| THE HAGUE | 07-06-1989 | SCARPONI U. |

EPO FORM 1503 03.82 (P0401)